# EUROPÄISCHE PATENTSCHRIFT

(11) **EP 1 399 138 B1**
(45) Veröffentlichungstag und Bekanntmachung des Hinweises auf die Patenterteilung: **08.02.2006**
(21) Anmeldenummer: 02743039.6
(22) Anmeldetag: 17.05.2002
(51) Int. Cl.: A61K 31/19

(54) **VERFAHREN ZUR HERSTELLUNG FESTER FORMULIERUNGEN VON NATRIUM-3-HYDROXY-3-METHYLBUTYRAT**
METHOD FOR THE PRODUCTION OF SOLID FORMULATIONS OF SODIUM 3-HYDROXY-3-METHYLBUTYRATE
PROCEDE DE PRODUCTION DE COMPOSITIONS SOLIDES DE 3-HYDROXY-3-METHYLBUTYRATE DE SODIUM

(30) Priorität: 18.05.2001 EP 01112236
(43) Veröffentlichungstag der Anmeldung: 24.03.2004
(73) Patentinhaber: Lonza AG, 4052 Basel (CH)
(72) Erfinder: HEYL-FRANK, Brigitta, CH-3930 Visp (CH); IRLE, Heike, CH-3931 Lalden (CH); PIANZOLA, Daniel, CH-3902 Brig-Glis (CH); ZACHER, Uwe, CH-3900 Brig (CH); JACKSON, Barry, CH-3902 Brig-Glis (CH)
(86) Internationale Anmeldenummer: PCT/EP2002/005435
(87) Internationale Veröffentlichungsnummer: WO 2002/094255

(56) Entgegenhaltungen:
- WO-A-94/06417
- WO-A-94/14429
- WO-A-98/34897
- US-A- 6 090 978
- S. NISSEN ET AL.: "beta-Hydroxy-beta-methylbutyrate (HMB) supplementation in humans is safe and may decrease cardiovascular risk factors" THE JOURNAL OF NUTRITION, Bd. 130, Nr. 8, 2000, Seiten 1937-1945, XP002215248

## Beschreibung

Die Erfindung betrifft ein Verfahren zur Herstellung fester Formulierungen des Natriumsalzes von 3-Hydroxy-3-methylbuttersäure (β-Hydroxy-β-methylbuttersäure, HMB) der Formel

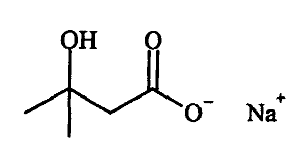

3-Hydroxy-3-methylbuttersäure ist ein Wirkstoff, der als Medikament und Nahrungszusatz für Mensch und Tier eingesetzt wird, beispielsweise zur Senkung des Cholesteringehaltes im Blut (WO-A-94/06417) oder zur Verminderung der Stickstoffausscheidung, insbesondere bei kranken und/oder alten Menschen (WO-A-94/14429). Üblicherweise wird hierzu nicht die freie Säure, sondern ein physiologisch verträgliches Salz, beispielsweise das Calciumsalz, eingesetzt. Aus wirtschaftlichen Gründen wäre es insbesondere für Anwendungen in der Tierernährung oder Veterinärmedizin wünschenswert, auch das Natriumsalz einsetzen zu können. Dieses wurde zwar schon mehrfach in der Literatur erwähnt, es ist jedoch kein technisches Verfahren zu seiner Herstellung in fester Form beschrieben und es hat sich gezeigt, dass es nicht lagerstabil und hygroskopisch und deshalb schlecht zu verarbeiten ist.

Aufgabe der vorliegenden Erfindung war daher, ein einfaches und wirtschaftliches Verfahren zur Herstellung einer lagerstabilen festen (pulverförmigen) und wenig hygroskopischen Form von Natrium-3-hydroxy-3-methylbutyrat bereitzustellen.

Erfindungsgemäss wird diese Aufgabe durch das Verfahren nach Patentanspruch 1 gelöst.

Es wurde gefunden, dass eine durch Hydrolyse von 4,4-Dimethyloxetan-2-on mit wässrigem Natriumhydroxid erhältliche Lösung von Natrium-3-hydroxy-3-methylbutyrat, gegebenenfalls nach Aufkonzentrieren, durch Aufziehen auf synthetische Kieselsäure und gegebenenfalls anschliessende Trocknung in eine wenig hygroskopische, rieselfähige und gut handhabbare Form gebracht werden kann.

Als synthetische Kieselsäure eignen sich insbesondere die handelsüblichen Fällungskieselsäuren, wie sie beispielsweise von Degussa unter der Bezeichnung Sipernat® im Handel erhältlich sind.

Vorzugsweise wird die Lösung von Natrium-3-hydroxy-3-methylbutyrat durch Abdestillieren von Wasser unter vermindertem Druck auf eine Konzentration von wenigstens 50 Gew.%, besonders bevorzugt 70 bis 80 Gew.%, gebracht. Bei den letztgenannten Konzentrationen wird die Lösung zweckmässig auf einer Temperatur von 60-65 °C gehalten, um ein Auskristallisieren zu verhindern. Das Aufkonzentrieren kann im Labormassstab beispielsweise in einer gewöhnlichen Destillationsapparatur oder in technischem Massstab beispielsweise in einem Dünnschichtverdampfer erfolgen. Es ist aber auch möglich, die bei der Synthese anfallenden Lösungen mit Gehalten von beispielsweise 20 bis 30 Gew.% direkt einzusetzen.

Das Aufziehen der Lösung von Natrium-3-hydroxy-3-methylbutyrat kann in einer üblichen Mischvorrichtung erfolgen, vorzugsweise wird hierzu ein schonender Mischertyp wie beispielsweise ein Trommel-, Pflugschar- oder Konusmischer, ein Sprühmischer oder auch ein (langsamlaufender) Intensivmischer eingesetzt. Ein zu heftiger Mischvorgang kann überraschenderweise zu Klumpenbildung fuhren.

Vorzugsweise erfolgt das Aufziehen dergestalt, dass die synthetische Kieselsäure vorgelegt und die Lösung von Natrium-3-hydroxy-3-methylbutyrat zudosiert wird.

Zur Verringerung des Wassergehalts im fertigen Produkt kann dieses noch auf an sich bekannte Weise getrocknet werden. Es werden aber auch ohne Trocknung selbst bei Wassergehalten von bis zu 30 Gew.% noch rieselfähige und äusserlich trockene Produkte erhalten.

Zur weiteren Verbesserung der Fliessfähigkeit kann dem erfindungsgemäss hergestellten Produkt gegebenenfalls noch hydrophobe Kieselsäure (z. B. Sipernat® D17) oder ein anderer Fliessverbesserer zugesetzt werden. Hierbei können beispielsweise Mengen von 0,2 bis 0,4 Gew.%, bezogen auf die Trockensubstanz, hydrophober Kieselsäure gegen Ende des Mischvorgangs zugesetzt und für kurze Zeit (z. B. 30 min) untergemischt werden.

Die nach dem erfindungsgemässen Verfahren erhältlichen festen Zusammensetzungen, die Natrium-3-hydroxy-3-methylbutyrat und synthetische Kieselsäure enthalten, sind ebenfalls Gegenstand der Erfindung.

Vorzugsweise enthalten die erfindungsgemässen Zusammensetzungen, bezogen auf die Trockensubstanz, wenigstens 25 Gew.% Natrium-3-hydroxy-3-methylbutyrat, besonders bevorzugt 25 bis 50 Gew.%.

Die folgenden Beispiele verdeutlichen die Ausführung der Erfindung, ohne dass darin eine Einschränkung zu sehen ist.

### Beispiel 1

### Herstellung einer Lösung von Natrium-3-hydroxy-3-methylbutyrat

Eine Lösung von Natriumhydroxid (127,3 g; 3,15 mol) in Wasser (1,51) wurde auf 0 °C gekühlt. Dann wurde rohes 4,4-Dimethyloxetan-2-on (= β-Isovalerolacton; 336,7 g; 3 mol; erhalten durch Umsetzung von Keten mit Aceton) innerhalb von ca. 2¼ h unter kräftigem Rühren zugetropft, wobei die Temperatur zwischen 1,5 und 2 °C gehalten wurde. Das Reaktionsgemisch wurde weitere 15 min bei 0 °C gerührt und dann noch 40 min auf 22 °C erwärmt. Anschliessend wurden innerhalb von ca. 3 h bei 300 mbar 415 g Wasser abdestilliert. Auf diese Weise wurden 1533 g einer Lösung mit ca. 27,5 Gew.% Natrium-3-hydroxy-3-methylbutyrat erhalten.

### Beispiel 2

### Herstellung der festen Formulierung

Die Lösung aus Beispiel 1 wurde bei 50 mbar und 56 °C auf 57,5 Gew.% aufkonzentriert. In einem Eirich-Intensivmischer (Typ R02) wurden 50 g synthetische Kieselsäure (Sipernat® 50, Degussa AG) vorgelegt Die Tellerdrehung wurde auf Stufe 2 eingestellt (der rotierende Messerkopf war ausser Betrieb) und innerhalb von 1 h wurden 100 g der Lösung über einen Schlauch zudosiert. Danach wurde der Mischvorgang noch für ½ h fortgesetzt. Es wurde ein homogenes, klumpenfreies, rieselfähiges und äusserlich trockenes Produkt erhalten.

## Patentansprüche

1. Verfahren zur Herstellung fester Formulierungen von Natrium-3-hydroxy-3-methylbutyrat der Formel 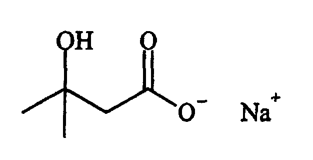
**dadurch gekennzeichnet, dass** in einem ersten Verfahrensschritt 4,4-Dimethyloxetan-2-on mit wässrigem Natriumhydroxid zu einer Lösung von Natrium-3-hydroxy-3-methylbutyrat umgesetzt, diese Lösung, gegebenenfalls nach Aufkonzentrieren, in einem weiteren Verfahrensschritt auf synthetische Kieselsäure aufgezogen und das so erhaltene Produkt gegebenenfalls getrocknet wird.

2. Verfahren nach Anspruch 1, **dadurch gekennzeichnet, dass** die Lösung von Natrium-3-hydroxy-3-methylbutyrat unter vermindertem Druck auf eine Konzentration von wenigstens 50 Gew.% aufkonzentriert wird.

3. Verfahren nach Anspruch 1 oder 2, **dadurch gekennzeichnet, dass** das Aufziehen auf synthetische Kieselsäure in einem Trommel-, Pflugschar- oder Konusmischer oder einem Intensivmischer erfolgt.

4. Verfahren nach einem der Ansprüche 1 bis 3, **dadurch gekennzeichnet, dass** das Aufziehen auf die synthetische Kieselsäure **dadurch** erfolgt, dass die synthetische Kieselsäure vorgelegt und die Lösung von Natrium-3-hydroxy-3-methylbutyrat zudosiert wird.

5. Verfahren nach einem der Ansprüche 1 bis 4, **dadurch gekennzeichnet, dass** zur Verbesserung der Fliessfähigkeit noch hydrophobe Kieselsäure zugesetzt wird.

6. Feste Zusammensetzung enthaltend Natrium-3-hydroxy-3-methylbutyrat und synthetische Kieselsäure, erhältlich nach dem Verfahren gemäss Ansprüchen 1 bis 5.

7. Feste Zusammensetzung nach Anspruch 6, **dadurch gekennzeichnet, dass** der Gehalt an Natrium-3-hydroxy-3-methylbutyrat, bezogen auf die Trockensubstanz, wenigstens 25 Gew.%, vorzugsweise 25 bis 50 Gew.%, beträgt.

## Claims

1. A process for preparing solid formulations of sodium 3-hydroxy-3-methylbutyrate of the formula 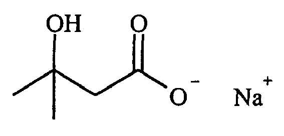
**characterized in that**, in a first process step, 4,4-dimethyloxetan-2-one is reacted with aqueous sodium hydroxide to form a solution of sodium 3-hydroxy-3-methylbutyrate, this solution, if appropriate after concentration, is applied, in a further process step, to synthetic silica, and the resultant product is, if appropriate, dried.

2. The process as claimed in claim 1, **characterized in that** the solution of sodium 3-hydroxy-3-methylbutyrate is concentrated under reduced pressure to a concentration of at least 50% by weight.

3. The process as claimed in claim 1 or 2, **characterized in that** the application to synthetic silica is performed in a drum mixer, plowshare mixer or cone mixer, or an intensive mixer.

4. The process as claimed in one of claims 1 to 3, **characterized in that** the application to the synthetic silica is carried out by first introducing the synthetic silica and then adding the solution of sodium 3-hydroxy-3-methylbutyrate.

5. The process as claimed in one of claims 1 to 4, **characterized in that**, to improve the flowability, hydrophobic silica is further added.

6. A solid composition comprising sodium 3-hydroxy-3-methylbutyrate and synthetic silica, obtainable by the process according to claims 1 to 5.

7. The solid composition as claimed in claim 6, **characterized in that** the content of sodium 3-hydroxy-3-methylbutyrate, based on the dry matter, is at least 25% by weight, preferably from 25 to 50% by weight.

## Revendications

1. Procédé de préparation de formulations solides de 3-hydroxy-3-méthylbutyrate de sodium de la formule 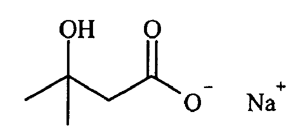
**caractérisé en ce que**, dans une première étape du procédé, on fait réagir de la 4,4-diméthyloxétan-2-one avec de l'hydroxyde de sodium aqueux pour former une solution de 3-hydroxy-3-méthylbutyrate de sodium, **en ce que** cette solution, éventuellement après concentration, est absorbée dans une deuxième étape du procédé sur de l'acide silicique synthétique et **en ce que** le produit ainsi obtenu est éventuellement séché.

2. Procédé selon la revendication 1, **caractérisé en ce que** la solution de 3-hydroxy-3-méthylbutyrate de sodium est concentrée sous pression réduite à une concentration d'au moins 50% en poids.

3. Procédé selon la revendication 1 ou 2, **caractérisé en ce que** l'absorption sur de l'acide silicique synthétique a lieu dans un mélangeur à tambour, à socs ou à cône ou dans un mélangeur intensif.

4. Procédé selon l'une quelconque des revendications 1 à 3, **caractérisé en ce que** l'absorption sur l'acide silicique synthétique est entreprise **en ce que** l'on charge l'acide silicique synthétique et l'on y ajoute la solution de 3-hydroxy-3-méthylbutyrate de sodium.

5. Procédé selon l'une quelconque des revendications 1 à 4, **caractérisé en ce que** l'on ajoute encore de l'acide silicique hydrophobe pour améliorer la fluidité.

6. Composition solide contenant du 3-hydroxy-3-méthylbutyrate de sodium et de l'acide silicique synthétique, que l'on peut obtenir par le procédé selon les revendications 1 à 5.

7. Composition solide selon la revendication 6, **caractérisée en ce que** la teneur en 3-hydroxy-3-méthylbutyrate de sodium, par rapport à la matière sèche, est d'au moins 25% en poids, de préférence de 25 à 50% en poids.
